(19) **Europäisches Patentamt**

**European Patent Office**

**Office européen des brevets**

(11) **EP 0 488 078 B1**

(12) **EUROPÄISCHE PATENTSCHRIFT**

(45) Veröffentlichungstag und Bekanntmachung des Hinweises auf die Patenterteilung:
**07.02.1996 Patentblatt 1996/06**

(51) Int. Cl.$^6$: **A23L 1/305**, A61K 38/00

(21) Anmeldenummer: **91119951.1**

(22) Anmeldetag: **22.11.1991**

(54) **Phenylalaninfreies Diätetikum für juvenile und adulte Personen mit Phenylketonurie**

Diet free of phenylalanin for juveniles and adults affected with phenylketonurea

Diète exempt de phénylalanine pour personnes adolescentes et adultes ayant une maladie phénylcétoneuréa

(84) Benannte Vertragsstaaten:
**AT BE CH DK ES FR GB GR IT LI LU NL SE**

(30) Priorität: **24.11.1990 DE 4037447**

(43) Veröffentlichungstag der Anmeldung:
**03.06.1992 Patentblatt 1992/23**

(73) Patentinhaber: **MILUPA AKTIENGESELLSCHAFT**
**D-61381 Friedrichsdorf (DE)**

(72) Erfinder:
• **Wachtel, Ursula, Dr.**
**W-6380 Bad Homburg (DE)**
• **Schweikhardt, Friedrich, Dr.**
**W-6382 Friedrichsdorf 2 (DE)**

• **Tesmer, Erhard, Dr.**
**W-6380 Bad Homburg (DE)**

(74) Vertreter: **Köster, Hajo, Dr. et al**
**D-82131 Gauting b. München (DE)**

(56) Entgegenhaltungen:
EP-A- 0 259 167          FR-A- 2 605 854
US-A- 4 209 351          US-A- 4 252 822

• **PATENT ABSTRACTS OF JAPAN Band 11, Nr. 63 (C- 406)(2510), 26. Februar 1987; & JP - A - 61225127 (SNOWBRAND MILK PRODUCT CO LTD) 06.10.1986**

## Beschreibung

Die Erfindung betrifft ein phenylalaninfreies Diätetikum auf Basis von Aminosäuren für Personen, insbesondere juvenile und adulte Personen, die unter einer Phenylketonurie leiden, sowie ein Verfahren zur Herstellung eines phenyialaninfreien Diätetikums.

Die Phenylketonurie (nachstehend PKU genannt) gehört zu den genetisch bedingten Erkrankungen und stellt eine Stoffwechselstörung dar.

Phenylalanin wird unter normalen Ernährungsbedingungen mit allen Proteinen tierischer und pflanzlicher Herkunft aufgenommen. Nach der Verdauung der nativen Proteine und Resorption der Spaltprodukte gelangt Phenylalanin über den Aminosäuren-Pool in die Leber und wird dort in einer irreversiblen Reaktion durch das Enzym Phenylalaninhydroxylase zu Tyrosin hydroxyliert. Bei Säuglingen und Kleinkindern wird in Folge des raschen Wachstums ein Teil des Phenylalanins für die körpereigene Proteinsynthese benötigt.

Als Folge der Stoffwechselstörung häuft sich bei den erkrankten Personen bzw. Patienten das Phenylalanin im Körper an, so daß sich dessen Gehalt im Blut und im Gewebe weit über den Normalbereich hinaus erhöht.

Der erhöhte Phenylalaninspiegel im Plasma führt zu einer Beeinträchtigung zahlreicher Stoffwechselvorgänge im Gehirn. Wird die PKU nicht behandelt, kommt es bei Kindern zu einer Hirnreifestörung mit geistigem Entwicklungsrückstand in unterschiedlicher Ausprägung.

Um bei einem unter PKU leidenden Kind eine normale geistige und körperliche Entwicklung zu erzielen, muß sein Phenylalaninspiegel im Plasma mit einer phenylalaninarmen Diät auf Normalwerte gesenkt und stabilisiert werden.

Dazu bekommen derartige Kinder eine Nahrung, die eine beschränkte Menge an natürlichem Eiweiß und gerade soviel Phenylalanin enthält, wie der kindliche Körper zum Aufbau von Eiweiß (Wachstum) benötigt. Für die Ernährung können daher nur solche Lebensmittel eingesetzt werden, die von Natur aus einen niedrigen Eiweißgehalt und damit auch einen niedrigen Phenylalaningehalt besitzen.

Mit einer derartigen phenylalaninarmen Ernährung allein würde den Kindern jedoch zu wenig von allen anderen, für das Leben ebenso wichtigen Aminosäuren zugeführt werden. Daher benötigen Kinder, die an der PKU leiden, zusätzlich zu einer phenylalaninarmen Diät eine Eiweißquelle, die zwar kein Phenylalanin, jedoch aber eine ausreichende Menge aller anderen Aminosäuren enthält.

Es sind bereits Spezialerzeugnisse bekannt, die aus Eiweißbausteinen, den Aminosäuren, zusammengesetzt sind, jedoch kein Phenylalanin enthalten. Diesen Mischungen sind zusätzlich Vitamine, Mineralstoffe und Spurenelemente einverleibt, da ein unter der PKU leidendes Kind diese Nährstoffe mit der phenylalaninarmen Ernährung allein nicht in ausreichenden Mengen erhalten würde.

Wegen der Störanfälligkeit des Hirnstoffwechsels durch einen erhöhten Phenylalaninspiegel im Plasma, auch nach Eintritt der Pubertät, raten heutzutage immer mehr Fachleute ihren Patienten, die phenylalaninarme Diät lebenslang beizubehalten.

Hinter den medizinischen Aspekten der Betreuung der an der PKU chronisch erkrankten Kinder dürfen die menschlichen Probleme, die sich insbesondere aus dem Zwang zur Einhaltung einer Diät ergeben, nicht übersehen werden.

Ein diesbezüglich schwieriges Lebensalter ist insbesondere die Adoleszenz, in der die Behandlungsbedürftigkeit der PKU von den jugendlichen Patienten häufig verleugnet und verdrängt wird. Es hat sich jedoch gezeigt, daß sich die mangelnde Einhaltung der Diät bzw. deren Ablehnung ungünstig auf die Entwicklung der Patienten auswirkt, während die intellektuelle, neurologische und psychomotorische Entwicklung um so günstiger verläuft, je niedriger der Phenylalaninspiegel über die Dauer der Behandlung ist.

Bisher wurde nun die Auffassung vertreten, daß der Eiweißstoffwechsel die gleichzeitige Anwesenheit aller und somit auch der nicht essentiellen Aminosäuren erfordert. Aus diesem Grunde enthalten die bekannten und üblicherweise eingesetzten Spezialprodukte bzw. phenylalaninfreien Aminosäuremischungen für Säuglinge und Kinder mit ihrem hohen Wachstumsbedarf auch alle Aminosäuren mit Ausnahme des in seinem Stoffwechsel gestörten Phenylalanins.

In Abhängigkeit von der individuellen Stoffwechselsituation eines unter der PKU leidenden Patienten mußte dieser pro Tag 45 bis 70 g der bekannten phenylalaninfreien Aminosäurenmischung zu sich nehmen. Dies stellt eine erhebliche Stoffmenge dar, welche die Akzeptanz dieser bekannten Spezialprodukte durch die Patienten, insbesondere durch juvenile Patienten, stark negativ beeinflusst.

Es ist auch bereits versucht worden, durch die Gabe von einzelnen essentiellen Aminosäuren die durch den erhöhten Phenylalaninspiegel gestörten neuromotorischen Funktionen zu normalisieren. So empfiehlt die US-A-4 252 822 beispielsweise die Verabreichung eines Gemisches der einzelnen verzweigten Aminosäuren Valin, Isoleucin und Leucin. Eine derartige isolierte Verabreichung einzelner Aminosäuren führt jedoch zu Imbalancen im Serum, weshalb dieses Behandlungsprinzip weitgehend abgelehnt wird.

Auch ist es auch der EP-A-0 259 167 bekannt, verschiedene freie Aminosäuren in unterschiedlicher Zusammensetzung einem Nahrungsmittel für Athleten einzuverleiben.

Aufgabe der vorliegenden Erfindung ist es daher, ein phenylalaninfreies Diätetikum bzw. eine phenylalaninfreie Aminosäurenmischung bereitzustellen, die von den unter der PKU leidenden Patienten besser akzeptiert wird und den Anforderungen entspricht, die an ein lebenslang einzunehmende Diätetikum gestellt werden.

Gelöst wird diese Aufgabe durch das phenylalaninfreie Diätetikum gemäß Anspruch 1.

Es wurde nämlich überraschend gefunden, daß der tägliche Eiweißbedarf von jugendlichen und erwachsenen, unter der PKU leidenden Patienten mit einer Aminosäuremischung vollständig oder fast vollständig gedeckt werden kann, die vorwiegend oder ausschließlich nur die essentiellen Aminosäuren mit Ausnahme des Phenylalanins sowie L-Tyrosin und L-Histidin enthält. Bei diesen Patienten muß durch die Eiweißzufuhr kein Wachstumsbedarf mehr befriedigt werden. Es muß lediglich nur noch der Erhaltungsbedarf der Körpersubstanz abgedeckt werden.

Das erfindungsgemäße Diätetikum enthält daher an Aminosäuren zu mindestens 95% nur bestimmte Aminosäuren. Jedoch sind auch solche Diätetika als erfindungsgemäße Diätetika zu betrachten, die auch geringe Mengen an anderen Aminosäuren enthalten, die nicht zu den expressis verbis im Anspruch 1 aufgeführten Aminosäuren zählen. Vorzugsweise sind jedoch ausschließlich diese bestimmten bzw. namentlich aufgeführten Aminosäuren vorhanden.

Die erfindungsgemäß eingesetzten Aminosäuren liegen natürlich in der L-Form vor, auch wenn dies nicht immer angegeben ist.

Die Aminosäuren werden bis auf das Lysin vorzugsweise als freie Aminosäuren eingesetzt. Das Lysin liegt vorzugsweise als Lysinacetat vor. Die Aminosäuren können jedoch in jeder geeigneten Form und insbesondere in jeder für Lebensmittelzwecke zulässigen Form vorliegen. So können die Aminosäuren beispielsweise als Salze, Hydochloride, Hydrate, Glutamate, Acetate, Aspartate, Glutamate, Malate. etc vorliegen. Da das Gewicht und die Gewichtsmenge des erfindungsgemäßen Diätetikums jedoch möglichst gering sein soll, werden die Aminosäuren vorzugsweise - wie oben geschildert - als freie Aminosäuren eingesetzt. Auch Oligopeptide, die vorwiegend oder ausschließlich aus den erfindungsgemäß eingesetzten Aminosäuren aufgebaut sind, können Anwendung finden, sofern sie frei von Phenylalanin sind.

Die Angabe, daß die erfindungsgemäß eingesetzten Aminosäuren 95 % der insgesamt vorhandenen Aminosäuren ausmachen bzw. daß eine bestimmte Aminosäure einen gewissen %-Anteil bzw. Gew.-%-anteil ausmacht, bezieht sich auf das Gewicht bzw. den Gewichtsanteil der Aminosäure(n) in freier Form bzw. als freie Säure. Liegt eine Aminosäure nicht in freier Form sondern zum Beispiel in Form eines oben beschriebenen Derivats bzw. Oligopeptids vor, dann muß dies natürlich bei der Berechnung berücksichtigt werden. Es muß somit umgerechnet werden, welcher Menge an entsprechender freier Aminosäure die gegebene Menge an Aminosäurederivat bzw. an Oligopeptid entspricht. Auch die Summe der Aminosäuren wird auf Basis der Aminosäuren in freier Form berechnet.

Das erfindungsgemäße Diätetikum ist primär für Jugendliche (vom älteren Schulkind an) und erwachsene Patienten gedacht, kann jedoch auch für Kinder Anwendung finden, sofern die für die Deckung des Wachstumsbedarfs erforderlichen weiteren Aminosäuren zusätzlich zugeführt werden.

Für die betroffen, unter der PKU leidenden Patienten, ergibt sich bei Verwendung des erfindungsgemäßen Diätetikums der große Vorteil, daß sie nur die Hälfte der Tagesdosis verzehren müssen, verglichen mit der bisher bekannten Aminosäurenmischung. Dies kann zu einer besseren "compliance" der Patienten führen und eine günstigere intellektuelle neurologische und psychomotorische Entwicklung bewirken.

Eine mehrjährige engmaschige Verlaufskontrolle von insgesamt 10 jugendlichen und erwachsenen, an der PKU leidenden Patienten hat ergeben, daß der tägliche Eiweißbedarf durch die erfindungsgemäße phenylalaninfreie Mischung bestimmter Aminosäuren zusammen mit geringen Mengen an natürlichem Protein gedeckt werden kann.

Selbst bei einer Eiweißzufuhr von lediglich 0,65 g pro kg Körpergewicht und Tag ergibt sich kein klinischer Hinweis auf einen Katabolismus. Diese Menge liegt deutlich unter den Richtwerten, die für Jugendliche und Erwachsene empfohlen werden, nämlich 0,9 g pro kg Körpergewicht und Tag laut der Weltgesundheitsorganisation (1985) bzw. 45 bis 50 g pro Tag laut der Deutschen Gesellschaft für Ernährung (1989).

Der einzelne Patient muß in Abhängigkeit von seiner individuellen Stoffwechselsituation eine Tagesmenge von 8 bis 22 g des erfindungsgemäßen phenylalaninfreien Diätetikums auf Basis von bestimmten Aminosäuren zu sich nehmen, verglichen mit 45 bis 70 g der bisher bekannten Aminosäurenmischung. Als Folge dieser außerordentlichen Vereinfachung der Diät hat bisher keiner der Patienten die Behandlung aufgegeben.

Nach einer bevorzugten Ausführungsform erhält das erfindungsgemäße Diätetikum neben den Aminosäuren zusätzlich Zucker, Mineralstoffe, Spurenelemente und/oder Vitamine sowie gegebenenfalls insbesondere lebensmittelrechtlich zulässige Monoglyceride, Diglyceride und/oder Triglyceride.

Die Aminosäuren sind vorzugsweise in folgenden Anteilen, ausgedrückt in Gew.-% und und berechnet als freie Säure sowie bezogen auf die Summe der freien Aminosäuren, vorhanden:

| Aminosäure | Gew.-% |
|---|---|
| L-Lysin | 14,5 |
| L-Histidin | 5,0 |
| L-Isoleucin | 11,9 |
| L-Leucin | 20,2 |
| L-Methionin | 4,9 |
| L-Threonin | 9,5 |
| L-Valin | 14,4 |
| L-Tryptophan | 3,6 |
| L-Tyrosin | 16,0 |
|  | 100,00 |

Die Anteile der eingesetzten Aminosäuren sind dabei so berechnet und aufeinander abgestellt, daß die Mischung möglichst optimal für die Abdeckung des Erhaltungsbedarfs ist.

Diese Anteile an den einzelnen Aminosäuren können jedoch in Abhängigkeit von der individuellen Stoffwechselsituation schwanken. So können die Anteile für Lysin, Isoleucin, Leucin, Valin und Tyrosin jeweils um bis zu ± 10 % und die Anteile für die übrigen Aminosäuren um bis zu ± 20 % von dem genannten Wert abweichen. Wird nun eine ( oder mehrere ) Aminosäure in einem großen Anteil eingesetzt, dann versteht es sich von selbst, daß eine ( oder mehrere ) andere Aminosäure in einem kleineren Anteil eingesetzt wird.

Ferner können die Anteile bzw. Gehalte an Tryptophan und Tyrosin in der erfindungsgemäßen Mischung in Abhängigkeit von der individuellen Neurotransmittersynthese gewählt werden.

Die für einen Patienten erforderliche Tagesmenge des erfindungsgemäßen Diätetikums wird zweckmäßigerweise in kleine Portionen von 5 bis 10 g aufgeteilt, wobei bis zu drei derartige Portionen pro Tag eingenommen werden. Die Einnahme derartig kleiner Volumina führt zu einer besseren Akzeptanz durch den Patienten. Die regelmäßige und über den Tag gleichmäßig verteilte Einnahme von derartigen Portionen führt außerdem zum Anabolismus im Eiweißstoffwechsel und zu einem gut ausgeglichenen Aminosäurenspiegel im Blut, so daß Imbalancen vermieden werden.

Bei der Herstellung von Aminosäurengemischen hat sich gezeigt, daß sich diese leicht entmischen, vor allem dann, wenn die Aminosäuren in feinkristalliner, gemahlener Form trocken vermischt werden. Aminosäurengemische, die mit Zuckerzusätzen versetzt wurden, neigen zudem zu Bräunungen und sind schlecht haltbar.

Erfindungsgemäß wird daher auch ein Verfahren zur Herstellung eines phenylalaninfreien Diätetikums auf Basis von Aminosäuren bereitgestellt.

Es hat sich nämlich überraschend gezeigt, daß die erfindungsgemäß hergestellte Aminosäurenmischung, die gegebenenfalls auch Mineralstoffe, Zucker, Spurenelemente, Vitamine und Monoglyceride, Diglyceride bzw. Triglyceride enthalten kann, sehr stabil ist und sich nicht entmischt, wenn die Bestandteile dieser Mischung in Wasser zusammen gelöst und anschließend sprühgetrocknet werden. Ein derart hergestelltes Produkt ist rieselfähig gut dosierbar und in mit $N_2/CO_2$-begasten Dosen oder in Vakuumpackungen 2 bis 3 Jahre lang haltbar. Auch in Aluminiumverbundfolie ist das Produkt sehr gut haltbar.

Zur Vermeidung von Bräunungsreaktionen werden die wesentlichen Komponenten des phenylalaninfreien Diätetikums in kaltem Wasser gelöst. Dazu werden zweckmäßigerweise zunächst alle Mineralstoffe gelöst. Bei der Auflösung der einzelnen Aminosäuren wird vorzugsweise eine bestimmte Reihenfolge der Zugabe der einzelnen Aminosäuren eingehalten.

Zweckmäßigerweise kontrolliert man den pH-Wert des erhaltenen Ansatzes derart, daß dieser pH-Wert nicht zu stark und vorzugsweise nicht unter 5,5 bis 5,6 absinkt. Vorzugsweise stellt man den pH-Wert mit vorzugsweise Kaliumhydroxid oder Kaliumcarbonat auf 6,0 bis 6,2 ein bzw. sorgt dafür, daß der pH diesen Wert beibehält. In diesem pH-Bereich bleibt der Ansatz nämlich stabil.

Nach dem Lösen bzw. Dispergieren aller Aminosäuren und Mineral-stoffe wird der Ansatz erwärmt, vorzugsweise auf etwa 70 bis 80°C. Im Anschluß daran werden die restlichen Bestandteile des phenylalaninfreien Diätetikums gemäß den Angaben im Beispiel 1 zugegeben. Vorzugsweise setzt man auch Monoglyceride und/oder Diglyceride dazu, die sich als Hüll- und Gleitsubstanzen als sehr günstig erwiesen haben.

Nach Zugabe aller Bestandteile wird der erhaltene Ansatz homogenisiert, wodurch eine sehr intensive und feinkörnige Verteilung aller Komponenten bewirkt wird. Dies trägt dazu bei, daß nach der anschließenden Sprühtrocknung keine Entmischung der Bestandteile mehr erfolgt.

Durch den Zusatz von Monoglyceriden und/oder Diglyceriden wird außerdem die Benetzbarkeit des erhaltenen Pulvers beim Lösen in Wasser wesentlich verbessert. Die Pulvermischung bildet in Wasser eine homogene Dispersion.

Das erfindungsgemäße Verfahren führt zu einer sehr homogenen Verteilung aller Bestandteile. Eine Entmischung, auch nicht während der Lagerung und des Transports, findet nicht statt. Das erfindungsgemäß hergestellte Diätetikum ist völlig homogen, gut benetzbar und in Wasser dispergierbar sowie gut haltbar. Die Maillard-Reaktion bzw. Bräunung ist minimiert. Das erhaltene Produkt ist gut rieselfähig und dosierbar.

Beispiel 1

Zusammensetzung eines erfindungsgemäßen phenylalaninfreien Diätetikums in Gew.-%

| | |
|---|---|
| Monoglyceride | 1,00 % |
| Zucker | 1,85 % |
| Vanillin | 0,05 % |
| L-Lysinacetat | 15,20 % |
| L-Histidin | 3,70 % |
| L-Isoleucin | 8,80 % |
| L-Leucin | 14,90 % |
| L-Methionin | 3,60 % |
| L-Threonin | 7,00 % |
| L-Tryptophan | 2,70 % |
| L-Valin | 10,70 % |
| L-Tyrosin | 11,80 % |
| Mineralstoffmischung | 18,00 % |
| Vitaminmischung | 0,70 % |
| | 100,00 % |

Herstellung einer Charge von 100 kg des erfindungsgemäßen phenylalaninfreien Diätetikums.

Man legt 150 l kaltes Leitungswasser in einem heizbaren, mit einem Y-Strahlrührer ausgerüsteten Doppelmanteltank vor. Mittels einer Venturidüse gibt man nacheinander folgende Bestandteile zum kalten Wasser:
18,0 kg Mineralstoffmischung
3,7 kg L-Histidin
8,8 kg L-Isoleucin
14,9 kg L-Leucin
15,2 kg L-Lysinacetat
3,6 kg L-Methionin
7,0 kg L-Threonin
2,7 kg L-Tryptophan
10,7 kg L-Valin
11,8 kg L-Tyrosin
1,85 kg Zucker
Während des Einziehens der einzelnen Bestandteile mittels der Venturidüse rührt man den Ansatz gleichzeitig mit dem Y-Strahlrührer kräftig, bis eine homogene, klumpenfreie Dispersion vorliegt.

Im Anschluß daran erwärmt man den Ansatz auf 70 bis 75°C, gibt 1 kg Monoglyceride dazu und verteilt homogen. Danach löst man 50 g Vanillin und 700 g Vitaminmischung im Ansatz.

Den Ansatz erhitzt man danach etwa 15 min auf 75 bis 80°C. Im Anschluß führt man eine einstufige Homogenisierung mit 100 bar durch. Das erhaltene Konzentrat mit 40 % TS (Trockensubstanz) trocknet man auf einem Düsensprühturm zu einem Pulver. Die Eingangstemperatur des Turms beträgt 180 bis 185°C; die Ausgangstemperatur des Turms beträgt 85 bis 90°C.

Das erhaltene Pulver kann dann in Portionen zu je 8 g in Aluthenbeuteln verpackt werden.

**Patentansprüche**

1. Phenylalaninfreies Diätetikum auf Basis von Aminosäuren für Personen mit Phenylketonurie, insbesondere für juvenile und adulte Personen, wobei die Aminosäuren L-Histidin, L-Isoleucin, L-Valin, L-Threonin, L-Methionin, L-Leucin, L-Tryptophan, L-Tyrosin und L-Lysin mindestens 95 % der insgesamt vorhanden Aminosäuren ausmachen, erhältlich durch Sprühtrocknen einer diese Aminosäuren enthaltenden wäßrigen Lösung bzw. Dispersion.

2. Phenylalaninfreies Diätetikum nach Anspruch 1, dadurch **gekennzeichnet**, daß es an Aminosäuren ausschießlich L-Histidin, L-Isoleucin, L-Valin, L-Threonin, L-Methionin, L-Leucin, L-Tryptophan, L-Tyrosin und L-Lysin enthält.

3. Phenylalaninfreies Diätetikum nach Anspruch 1 oder 2, dadurch **gekennzeichnet**, daß es außerdem Zucker, Mineralstoffe, Spurenelemente und/oder Vitamine und gegebenenfalls Monoglyceride Diglyceride und/oder Triglyceride enthält.

4. Phenylalaninfreies Diätetikum nach Anspruch 2 oder 3, dadurch **gekennzeichnet**, daß es die nachstehenden Aminosäuren in folgenden Anteilen, berechnet als freie Aminosäure in Gew.-% und bezogen auf die Summe der Aminosäuren (berechnet in freier Form), wobei das L-Lysin insbesondere als L-Lysinacetat vorliegt, während die übrigen Aminosäuren insbesondere als freie Säuren vorhanden sind, enthält:

| Aminosäure | Gew.-% |
|---|---|
| L-Lysin | 14,5 ± 10 % |
| L-Histidin | 5,0 ± 20 % |
| L-Isoleucin | 11,9 ± 10 % |
| L-Leucin | 20,2 ± 10 % |
| L-Methionin | 4,9 ± 20 % |
| L-Threonin | 9,5 ± 20 % |
| L-Valin | 14,4 ± 10 % |
| L-Tryptophan | 3,6 ± 20 % |
| L-Tyrosin | 16,0 ± 10 % |
|  | 100,00 |

5. Verfahren zur Herstellung eines phenylalaninfreien Diätetikums nach einem der Ansprüche 1 bis 4, dadurch **gekennzeichnet**, daß man die Aminosäuren und die gegebenenfalls vorhandenen Mineralstoffe, Zucker, Spurenelemente, Vitamine, Monoglyceride, Diglyceride und/oderTriglyceride in Wasser löst bzw. dispergiert und dann spühtrocknet.

6. Verfahren nach Anspruch 5, dadurch **gekennzeichnet**, daß man die Mineralstoffe und die Aminosäuren und gegebenenfalls Zucker in kaltem Wasser, insbesondere von 3 bis 10 °C löst bzw. dispergiert, dann den Ansatz erwärmt, insbesondere auf 70 - 80 °C, und anschließend die restlichen Bestandteile hinzugibt.

7. Verfahren nach Anspruch 5 oder 6, dadurch **gekennzeichnet**, daß man aussschließlich folgende L-Aminosäuren in folgender Reihenfolge einsetzt bzw. zugibt: L-Histidin, L-Isoleucin, L-Leucin, L-Lysin, L-Methionin, L-Threonin, L-Tryptophan, L-Valin, L-Tyrosin.

8. Verfahren nach einem der Ansprüche 5 - 7, dadurch **gekennzeichnet**, daß man den nach dem Lösen aller Bestandteile erhaltenen Ansatz homogenisiert.

9. Verfahren nach einem der Ansprüche 5 - 8, dadurch **gekennzeichnet**, daß man den pH-Wert des Ansatzes nicht unter 5,5 absinken läßt und insbesondere bei 6,0 - 6,2 hält, insbesondere durch Zugabe von Kaliumhydroxid oder Kaliumcarbonat.

## Claims

1. Phenylalanine-free amino-acid based dietary product for persons with phenylketonuria, in particular for juvenile and adult persons, wherein the amino acids L-histidine, L-isoleucine, L-valine, L-threonine, L-methionine, L-leucine, L-tryptophan, L-tyrosine and L-lysine make up at least 95 % of the total amino acids present, obtainable by spray-drying of an aqueous solution or dispersion containing these amino acids.

2. Phenylalanine-free dietary product according to claim 1, characterised in that as amino acids it contains exclusively L-histidine, L-isoleucine, L-valine, L-threonine, L-methionine, L-leucine, L-tryptophan, L-tyrosine and L-lysine.

3. Phenylalanine-free dietary product according to claim 1 or 2, characterised in that it also contains sugar, mineral substances, trace elements and/or vitamins and optionally monoglycerides, diglycerides and/or triglycerides.

4. Phenylalanine-free dietary product according to claim 2 or 3, characterised in that it contains the following amino acids in the following proportions, calculated as free amino acids in wt % and based on the sum of the amino acids (calculated in free form), wherein the L-lysine is in particular present as L-lysine acetate, while the other amino acids are in particular present as free acids:

| Amino Acid | Wt % |
|---|---|
| L-lysine | 14.5 ± 10 % |
| L-histidine | 5.0 ± 20 % |
| L-isoleucine | 11.9 ± 10 % |
| L-leucine | 20.2 ± 10 % |
| L-methionine | 4.9 ± 20 % |
| L-threonine | 9.5 ± 20 % |
| L-valine | 14.4 ± 10 % |
| L-tryptophan | 3.6 ± 20 % |
| L-tyrosine | 16.0 ± 10 % |
|  | 100.00 |

5. Process for the preparation of a phenylalanine-free dietary product according to one of claims 1 to 4, characterised in that the amino acids and the mineral substances, sugar, trace elements, vitamins, monoglycerides, diglycerides and/or triglycerides optionally present are dissolved or dispersed in water and then is spray-dried.

6. Process according to claim 5, characterised in that the mineral substances and the amino acids and optionally sugar are dissolved or dispersed in cold water, especially from 3 to 10°C, then the mixture is heated, especially to 70-80°C, and next the remaining components are added.

7. Process according to claim 5 or 6, characterised in that exclusively the following amino acids in the following order are used or added: L-histidine, L-isoleucine, L-leucine, L-lysine, L-methionine, L-threonine, L-tryptophan, L-valine, L-tyrosine.

8. Process according to one of the claims 5-7, characterised in that the mixture obtained after solution of all components is homogenised.

9. Process according to one of the claims 5-8, characterised in that the pH value of the mixture is not allowed to fall below 5.5 and in particular is held at 6.0-6.2, in particular by addition of potassium hydroxide or potassium carbonate.

**Revendications**

1. Produit diététique sans phénylalanine à base d'acides aminés pour des personnes atteints de phénylcétonurie, plus particulièrement pour des adolescents et des personnes adultes, les acides aminés L-histidine, L-isoleucine, L-valine, L-thréonine, L-méthionine, L-leucine, L-tryptophane, L-tyrosine et L-lysine représentant au total au moins 95 % de tous les acides aminés présents, produit que l'on peut obtenir par séchage par dispersion d'une solution aqueuse, ou bien d'une dispersion contenant un de ces acides aminés.

2. Produit diététique sans phénylalanine selon la revendication 1, caractérisé en ce qu'il contient exclusivement les acides aminés L-histidine, L-isoleucine, L-valine, L-thréonine, L-méthionine, L-leucine, L-tryptophane, L-tyrosine et L-lysine.

3. Produit diététique sans phénylalanine selon la revendication 1 ou 2, caractérisé en ce qu'il contient en outre du sucre, des substances minérales, des oligoéléments et/ou des vitamines et éventuellement des monoglycérides, des diglycérides ou des triglycérides.

4. Produit diététique sans phénylalanine selon la revendication 2 ou 3, caractérisé en ce qu'il contient les acides aminés suivants en portions suivantes, calculées sous forme d'acides aminés libres en pourcentages en poids et par rapport à la somme des acides aminés (calculés sous forme libre), la lysine étant présente notamment sous forme d'acétate de L-lysine alors que les autres acides aminés sont présents notamment comme acides libres :

| Acide aminé | % en poids |
|---|---|
| L-Lysine | 14,5 ± 10 % |
| L-Histidine | 5,0 ± 20 % |
| L-Isoleucine | 11,9 ± 10 % |
| L-Leucine | 20,2 ± 10 % |
| L-Méthionine | 4,9 ± 20 % |
| L-Thréonine | 9,5 ± 20 % |
| L-Valine | 14,4 ± 10 % |
| L-Tryptophane | 3,6 ± 20 % |
| L-Tyrosine | 16,0 ± 10 % |
| | 100,0 |

5. Procédé pour la préparation d'un produit diététique sans phénylalanine selon l'une des revendications 1 à 4, caractérisé en ce qu'on dissout ou bien disperse dans l'eau les acides aminés et les substances minérales, les sucres, les oligo-éléments, les vitamines, les monoglycérides, les diglycérides et/ou les triglycérides éventuellement présents, et ensuite on sèche par dispersion.

6. Procédé selon la revendication 5, caractérisé en ce qu'on dissout ou bien disperse les substances minérales et les acides aminés et éventuellement le sucre dans l'eau froide, notamment à une température de 3 à 10°C, puis on chauffe la charge notamment à 70 à 80°C et ensuite on ajoute les autres constituants.

7. Procédé selon la revendication 5 ou 6, caractérisé en ce qu'on utilise, ou bien on ajoute, exclusivement les acides L-aminés suivants, l'un après l'autre comme suit : L-histidine, L-isoleucine, L-leucine, L-lysine, L-méthionine, L-thréonine, L-tryptophane, L-valine, L-tyrosine.

8. Procédé selon l'une des revendications 5 à 7, caractérisé en ce qu'on homogénéise la charge après dissolution de tous les constituants.

9. Procédé selon l'une des revendications 5 à 8, caractérisé en ce qu'on veille à ce que la valeur du pH de la charge ne descende pas au-dessous de 5,5 et notamment qu'elle se maintienne à une valeur de 6,0 à 6,2, notamment par addition d'hydroxyde de potassium ou de carbonate de potassium.